# EUROPEAN PATENT SPECIFICATION

(11) **EP 2 069 382 B1**
(45) Date of publication and mention of the grant of the patent: **23.03.2011**
(21) Application number: 07804526.7
(22) Date of filing: 13.09.2007
(51) Int. Cl.: C07J 1/00

(54) **PROCESS FOR THE SELECTIVE ISOLATION, PURIFICATION AND SEPARATION OF MONOHYDROXYLATED 3,17-DIKETO-STEROID COMPOUNDS**
VERFAHREN ZUR SELEKTIVEN ISOLIERUNG, AUFREINIGUNG UND AUFTRENNUNG VON MONOHYDROXYLIERTEN 3,17-DIKETOSTEROIDVERBINDUNGEN
PROCÉDÉ D'ISOLATION SÉLECTIVE, DE PURIFICATION ET DE SÉPARATION DE COMPOSÉS 3,17-DICÉTO-STÉROÏDES MONOHYDROXYLÉS

(30) Priority: 15.09.2006 HU 0600727
(43) Date of publication of application: 17.06.2009
(73) Proprietor: Richter Gedeon Nyrt., 1103 Budapest (HU)
(72) Inventor: JAKSA, István, deceased (HU); NÉMETH, Sándor, 1103 Budapest (HU); KÖNCZÖL, Kálmán, 1134 Budapest (HU); TERDY, László, 1192 Budapest (HU); KOZMA, István, 1182 Budapest (HU); BARTA, Ferenc, 1098 Budapest (HU)
(74) Representative: HOFFMANN EITLE
(86) International application number: PCT/HU2007/000084
(87) International publication number: WO 2008/032131

(56) References cited:
- WO-A-2004/014934
- US-A- 4 035 236
- US-A- 4 036 695

## Description

The invention relates to a process for the selective isolation, purification and separation of monohydroxylated 3,17-diketo-steroid compounds from a solution obtained preferably by microbiological way containing a mixture of steroid compounds via selective extraction, purification and selective crystallization of the steroid compounds.

The monohydroxylated 3,17-diketo-steroid compounds, for example 13β-methyl-11α-hydroxy-4-gonene-3,17-dione, 13β-ethyl-15α-hydroxy-4-gonene-3,17-dione, 15α-hydroxy-4-androstene-3,17-dione and 9α-hydroxy-4-androstene-3,17-dione, as well as 6ξ-hydroxymethyl-1,4-androstadiene-3,17-dione (which is a mixture of 6α and 6ß stereoisomers) are valuable key intermediates in the chemical synthesis of active ingredients such as desogestrel, gestodene, drospirenone as well as hydrocortisone, prednisolone and exemestane.

In the industrial processes the microbiological hydroxylation is a well-tried practice, as in many cases only a special microorganism is able to hydroxylate a given molecule in the desired position. In most of the cases the chemical synthesis (if it is realizable) for solving this problem is very complicated, expensive, the yields are low and many by-products are formed. The microbiological hydroxylation in the practice of the pharmaceutical industry is carried out the following way: first a microorganism is selected which is able to hydroxylate a given molecule in the desired position by screening, then those optimal parameters (components of the nutrient, pH, temperature, dissolved oxygen, etc.) are determined, which should be kept to carry out the bioconversion with good conversion.

In the end of the above mentioned complicated procedure a large volume of fermentation broth is formed and the desired product, in our case the monohydroxylated steroid compound, should be isolated from this solution. The fermentation broth is heterogeneous both from physical and chemical point of view, it contains microbial cells, as well as the cellular components liberating from microbial cells, the remaining substrate, the remaining components of the nutrient, the metabolism products, the formed product and by-products, antifoam agents, detergents etc. The concentration of the product in the fermentation broth is usually between 1-10 g/dm³, therefore the fermentation broth is a "diluted solution" containing a large amount of impurities and naturally the desired product in the highest concentration.

As it can be seen the down-stream processing of the fermentation broths - the isolation of the desired product - is a very complicated procedure. The desired product and the remaining components of the nutrient, the metabolism products of the microorganism, the additives (antifoam agents, detergents), the remaining substrate, the formed by-products and other impurities should be separated. The situation is even more complicated as usually the solubility of steroid compounds in water is very low, therefore the formed product is partly in solution, partly precipitated on the surface of the biomass. In case of using higher concentration of the substrate the desired product should be isolated from two different places: from the filtrate of the fermentation broth and from the surface of the biomass.

Several attempts were made for the solution of the above mentioned problems. For example according to the Japanese patent 54-105294 in order to separate steroid mixtures the hydroxyl groups of steroid compounds are acylated then purified by column chromatography using silicagel or aluminum oxide and finally a fractionated crystallization yields the desired product. The publication does not contain numerical data concerning the effectiveness of the procedure, but the down-stream processing of an industrial scale acylation - using pyridine as solvent - may cause many problems. In this several-step procedure the operational loss of the product is necessarily high, so industrial application of this procedure is not economical.

The EP 245,985 Patent Specification describes the isolation of biologically active compounds on laboratory scale. According to the procedure the buffered solution of water soluble biologically active compounds is extracted with an appropriate special solvent which is immiscible with water (for example: polyethylene glycol) in the presence of micro-granulated (0.01-10 µm) adsorbent containing functional group. The product reversibly binds to the adsorbent from where after carrying out the separation processes of the phases can be recovered by known methods. Industrial realizability of this process is very doubtful.

The Hungarian Patent Specification 192,668 describes the microbiological synthesis of 15α-hydroxy-4-androstene-3,17-dione. During the process the bioconversion is carried out by the fungi *Penicillium melinii* or *Fusarium oxysporum f. vasinfectum.* The specification shortly describes the isolation method of steroid compounds as well, according to which the fermentation broth is extracted three times with isobutyl methyl ketone (total extraction), the combined extracts are concentrated on film evaporator and evaporated to dryness on rotary evaporator using water bath. The residue is washed with hexane in order to remove the antifoam agent and finally the steroid compounds are crystallized from isobutyl methyl ketone. The disadvantage of this process is that it yields only crude crystals. The description does not give any instruction about the further purification of the product and separation of the formed steroid by-products and the remaining substrate.

According to the process published in the Hungarian Patent Specification 217, 624 hydroxylated steroids are isolated from low concentration (1 g/dm³) filtrate of fermentation broth using macro-reticular adsorption resins and gradient elution. The ratio of the adsorbate and the adsorbent is 1:20, that is 20 g resin is needed to bind 1 g steroid. The limit of the process is that in case of higher concentration (6-10 g/dm³) of the substrate, on industrial scale (20-50 m³) high amount of adsorbent should be used, therefore both the packing and the equipment would be expensive, the process would not be economical.

US Pat. 4,036,695 describes a process for the preparation of estrene-3,17-dione compounds by fermenting an 18-alkyl-4-estrene-3,17-dione with a fungal culture of the genus Penicillium or Fusarium. When the conversion is complete, the fungus mycelium is filtered off and the culture broth is extracted with methyl isobutyl ketone. The organic extract solutions are combined and evaporated. The brown-crystalline residue is purified and recrystallized to yield pure 15α-hydroxy-18-methyl-4-estrene-3,17-dione derivative.

As it was mentioned earlier different hydroxylated steroid compounds, for example 13β-ethyl-11α-hydroxy-4-gonene-3,17-dione, 13β-ethyl-15α-hydroxy-4-gonene-3,17-dione, 15α-hydroxy-4-androstene-3,17-dione and 9α-hydroxy-4-androstene-3,17-dione, as well as 6ξ-hydroxymethyl-1,4-androstadiene-3,17-dione - our target compounds - are valuable key intermediates in the chemical synthesis of active ingredients such as desogestrel, gestodene, drospirenone as well as hydrocortisone, prednisolone and exemestane. The chemical syntheses are complicated, several-step processes therefore the operational loss of the product is necessarily high on industrial scale realization. Usually the use of 8-10 kg of the key-intermediate is required for the synthesis of 1 kg of the active ingredient. According to these facts the synthesis of hydroxylated steroid compounds should be carried out on large scale (occasionally several kg/batch).

The microbiological hydroxylation of steroid compounds can usually be carried out by 60-90 % bioconversion. The largest problem during the isolation of the product is the separation of the remaining substrate and the formed by-product, as in the next steps of the synthesis of the active intermediate only those products can be used the substrate content of which is lower than a given limit. This limit depends upon the product, usually it is between 1 and 2.5 %.

The above mentioned process can be carried out on laboratory and on pilot scale using silica gel or macro-reticular adsorption resins in the chromatographic separation. In the case of several hundred kilogram amount of substance none of this path can be used, as the amount of chromatographic packing and the solvents needed for this processes would be very large, the process would not be economical on industrial scale.

Our aim was to elaborate an economical, industrially applicable process, which eliminates the disadvantages of the known processes and enables the isolation of large amount of desired product in high purity and in good yield.

During our experiments we had to consider, that our desired compounds, the above mentioned hydroxylated steroid compounds, have very similar chemical, physical and physicochemical properties, their polarities are also only slightly different. This means, that all of these compounds behave very similarly during a given manipulation (for example dissolution, extraction, precipitation).

During our experiments we found, that the product formed in the fermentation process, the remaining substrate and the by-products - according to their solubility can be found both solved in the fermentation broth and precipitated on the surface of the biomass. That is exception, when the concentration of the added substrate is lower than 1.5 g/dm³, in this case the product remains in the solution. Accordingly the steroid compounds should be isolated both from the solution and the solid phase.

It was found that it is practical to remove the biomass from the fermentation broth by filtration, then the steroid compounds can be isolated from the filtrate with an organic solvent immiscible or partly miscible with water (aliphatic ester or aliphatic ketone). It was also found, that the non-steroid type impurities of biological origin can be selectively removed by treating the organic phase with an aqueous solution of basic character having appropriate ionic strength, then the crude product containing the steroid compounds can be obtained, after addition of water, by evaporation of the solvent. The steroid compounds are dissolved from the filtered biomass with aliphatic alcohols or ketones miscible with water using solid-liquid extraction and after evaporation of the solvent the crystalline crude product is obtained.

The crude crystalline products obtained from two places are mixed, dissolved in aliphatic alcohol or ketone miscible with water and the solution is treated with charcoal and/or perfil. The flocculant is filtered and the solvent is evaporated to yield the homogenous crude crystals. The so obtained homogenous crude crystals contain the product, 8-25 % remaining substrate depending on the bioconversion, as well as a few percent of other steroids. These compounds also have to be separated - as mentioned earlier - preferably not chromatographically.

The usual crystallization methods applied in the industrial practice (for example crystallization from organic solvent, from water or precipitation with water) were not successful, as to remove one unit of substrate caused the loss of 2-4 unit of product, that is the solubility of the product was better in these mixtures, than the solubility of the main impurity, the substrate.

During our experiments surprisingly it was found, that when the homogenous crude crystals were dissolved in chlorinated hydrocarbon and the product is precipitated by addition of aliphatic or aromatic hydrocarbon, the solubility ratio is changed. In this system the solubility of the apolar component (the substrate) is 10-60 times better, than the solubility of the polar compound (the product) depending on the amount of the substrate. Accordingly, the more apolar impurity can be removed by this selective crystallization without loosing large amount of product (the loss of product is less than 1 %). It is reasonable to dissolve the crude crystalline material in chlorinated hydrocarbon and to precipitate the product with aliphatic or aromatic hydrocarbon. In this case the main part of the substrate can be found in the mother liquor. If the bioconversion is low (20-30 % remaining substrate) the desired quality can be reached by repeating the above process.

According to the above mentioned facts, the present invention relates to a process for the selective isolation, purification and separation of monohydroxylated 3,17-diketo-steroid compounds from a solution obtained by microbiological hydroxylation containing a mixture of steroid compounds via selective extraction, purification and selective crystallization of the steroid compounds, which consists of the following steps:
a) removing the biomass from the fermentation broth, in given case after treatment with phosphoric acid, by filtration or centrifugation, isolating the steroid compounds from the filtrate with an aliphatic ketone or ester of an aliphatic alcohol, selectively removing the non-steroid type impurities from the organic phase by treatment with an aqueous solution of basic character and obtaining the crude crystalline product containing the hydroxylated steroids by addition of water and evaporation of the solvent and
b) dissolving the steroid compounds from the surface of the filtered biomass obtained in step a) by treatment with C₁-C₄ aliphatic alcohols or ketones or the aqueous solution thereof and obtaining the crude crystalline product containing the hydroxylated steroids after evaporation of the solvent, then
c) dissolving the product obtained in step a) or in given case the mixture of the two crude products obtained in step a) and b) in C₁-C₄ aliphatic alcohol or ketone miscible with water or the aqueous solution thereof, decolorizing the solution and isolating the purified homogenous crude crystals after evaporation of the solvent and
d) recrystallizing the purified homogenous crude crystals obtained in step c) from a mixture of chlorinated hydrocarbon/aliphatic or cycloaliphatic hydrocarbon and isolating the pure product.

According to the present invention the aliphatic esters of step a) preferably is ethyl acetate, n-propyl acetate and the aliphatic ketones preferably is isobutyl methyl ketone.

The basicity and the ionic strength of the aqueous solution used to remove non-steroid impurities are adjusted by addition of appropriate amount of alkali metal or alkali earth metal hydroxides and/or basic salts thereof, such as carbonates, hydrogen carbonates, hydrogen phosphates, acetates and the like.

According to the present invention an organic solvent miscible with water can preferably be C₁-C₃ aliphatic alcohols, such as methanol, ethanol, as well as aliphatic ketones, such as acetone and the like.

In the selective crystallization step an organic solvent immiscible with water can preferably be chlorinated hydrocarbons, such as dichloro methane, dichloro ethane and the like, and aliphatic or aromatic hydrocarbons can be used as precipitating solvent, such as n-hexane, n-octane, i-octane, 1-dodecane, cyclohexane, methyl-cyclohexane and the like.

The process according to the present invention can be used during the isolation of 13β-ethyl-11α-hydroxy-4-gonene-3,17-dione, 13β-ethyl-15α-hydroxy-4-gonene-3,17-dione, 15α-hydroxy-4-androstene-3,17-dione and 9α-hydroxy-4-androstene-3,17-dione, as well as 6ξ-hydroxymethyl-1,4-androstadiene-3,17-dione.

The advantages of the process of the present invention are as follows:
- realization thereof does not require expensive investment,
- effective, low-cost separation method,
- it provides high selectivity,
- loss of the product is very low,
- the process is fast, reproducible and easily accomplishable,
- the amount of applied solvents is low, for example 450-500 kg crude product can selectively be crystallized from 6 m³ solvent.

The present invention is illustrated by the following, non-limiting examples.

### Example 1

### Isolation of 15α-hydroxy-4-androstene-3,17-dione

The bioconversion is carried out by known method, for example according to the Hungarian Patent Specification 192,668 by fungus *Penicillium melinii,* in 900 dm³ useful volume. The amount of the added 4-androstene-3,17-dione substrate is 9 g/dm³, the bioconversion is 75%. After terminating the bioconversion the fermentation broth is analyzed and the pH of the fermentation broth is adjusted to 3.5-4.0 by addition of 10 % phosphoric acid in order to increase the efficacy of the filtration, 9 kg of perfil is added as filtration aid. The temperature of the fermentation broth is increased to 100 °C in order to destroy the microorganism. Then the temperature of the mixture is cooled to 40-45 °C and the biomass is filtered by C-10 type centrifuge fitted with technical canvas.

The weight of the filtered biomass is 36 kg.

The temperature of the filtrate is cooled to 20-25 °C and mixture is extracted with 500 dm³ of n-propyl acetate in an apparatus equipped with stirrer. After separating the phases the aqueous phase is repeatedly extracted with 250 dm³ of n-propyl acetate. The extraction is followed by TLC and HPLC techniques. The organic phases containing the steroid compounds are combined and concentrated to a volume of about 200 dm³ in a duplicator. The organic phase of decreased volume is extracted twice with 100 dm³ of 5 % sodium hydroxide solution in order to remove the non-steroid impurities. The aqueous phases are discarded after neutralization. 40 dm³ of water is added to the organic phase and the mixture is transferred to an apparatus, which can be heated or cooled and equipped with stirrer and the organic solvent is evaporated. The so obtained crude crystals (containing the steroid product) are filtered and dried.

The biomass is transferred to an apparatus, which can be heated or cooled and equipped with stirrer and 400 dm³ of 60 w/w % aqueous ethanol is added. The mixture is stirred for 30 minutes and the biomass is filtered by C-10 type centrifuge. Then the biomass is repeatedly extracted with 300 dm³ of 60 w/w % aqueous ethanol and separated by centrifugation. The filtered biomass is incinerated. The two filtrates are combined and the ethanol is distilled off in a duplicator. The residue is cooled to 20 °C and the precipitated steroids containing second crude product is filtered and dried.

The two crude crystalline products obtained by the above processes are transferred into an apparatus equipped with stirrer and dissolved in the mixture of 36 dm³ of acetone and 4 dm³ of water at 45-50 °C. Then 1 kg of Norit and 1 kg of perfil are added and the mixture is stirred for 30 min, then the flocculant is filtered and washed with a mixture of 1 dm³ of acetone and 3 dm³ of water. The filtrate and the washing are combined and concentrated to half volume in a duplicator. The residue is cooled to 10 °C, the precipitated purified crude crystals are filtered and dried.

The weight of the so obtained crystals are 6.4 kg, and according to HPLC the composition thereof is the following:

| | |
|---|---|
| 15α-hydroxy-4-androstene-3,17-dione | 85.0% |
| 4-androstene-3,17-dione | 10.9% |
| other steroids | 1.9% |

In order to separate the product and the substrate 64 dm³ of methyl cyclohexane is added to the above product and the water is removed from the mixture by azeotropic distillation. Then 25.6 dm³ (the volume is fourfold of the weight of the crystals) of dichloromethane is added to the mixture to obtain clear solution, which is clarified with 2 kg of aluminum oxide. The clarified solution is concentrated under atmospheric pressure to a volume of 70 dm³ and cooled to 25 °C. The precipitated crystals are filtered and dried.

The weight of the so obtained crystalline 15α-hydroxy-4-androstene-3,17-dione is 5.6 kg, and according to HPLC the composition thereof is the following (the yield is 89% calculated on the fermentation broth):

| | |
|---|---|
| 15α-hydroxy-4-androstene-3,17-dione | 96.2% |
| 4-androstene-3,17-dione | 1.4% |
| other steroids | 1.4% |

(When the last crystallization step is repeated - in given case - the amount of the substrate impurity can further be decreased.)

### Example 2

### Isolation of 13β-ethyl-15α-hydroxy-4-gonene-3,17-dione

The bioconversion is carried out by known method, for example according to the Hungarian patent 221 745 by fungus *Fusarium equiseti,* in 20 m³ useful volume. The amount of the added 13β-ethyl-4-gonene-3,17-dione substrate is 1.5 g/dm³, the bioconversion is 77%. After terminating the bioconversion the fermentation broth is analyzed and the major amount of the steroid compounds is found in the solution, precipitation on the surface of the biomass is only minimal.

The fermentation broth is filtered according to the method described in Example 1, with the difference that instead of centrifuge a filter press of 30 m² useful filtering surface is used. The weight of the filtered wet biomass is 500 kg (it is incinerated because of its low steroid content).

The filtrate of the fermentation broth is extracted with 4 m³ of ethyl acetate in a CINC type separator with countercurrent flow operational method. The raffinate is discarded and the organic phase is concentrated to a volume of 2 m³. The organic phase of decreased volume is extracted twice with 1 m³ of 5 % sodium hydroxide solution in order to remove the non-steroid impurities. The aqueous phases are discarded after neutralization. 600 dm³ of water is added to the organic phase and the mixture is transferred to an apparatus, which can be heated or cooled and equipped with stirrer and the organic solvent is evaporated. The so obtained crude, steroid containing crystals are filtered and dried.

The crude product is transferred into an apparatus equipped with stirrer and dissolved in the mixture of 180 dm³ of acetone and 360 dm³ of water at 45-50 °C. Then 4 kg of Norit charcoal and 4 kg of perfil are added and the mixture is stirred for 30 min, then the flocculants are filtered and washed with a mixture of 20 dm³ of acetone and 60 dm³ of water. The filtrate and the washing are combined and the organic solvent is evaporated in a duplicator. The residue is cooled to 10 °C, the precipitated purified crude crystals are filtered and dried.

The weight of the so obtained crystals is 21.5 kg, and according to HPLC the composition thereof is the following:

| | |
|---|---|
| 13β-ethyl-15α-hydroxy-4-gonene-3,17-dione | 94.6 % |
| 13β-ethyl-4-gonene-3,17-dione | 3.3% |
| other steroids | 2.6% |

In order to separate the product and the substrate the above crude product is dissolved in 54 dm³ (the volume is two and a half-fold of the weight of the crystals) of dichloromethane and 270 dm³ of methyl cyclohexane is added with stirring from a reservoir slowly over a period of about 1 h. The precipitated crystals are filtered and dried.

The weight of the so obtained 13β-ethyl-15α-hydroxy-4-gonene-3,17-dione is 20.8 kg, and according to HPLC the composition thereof is the following (the yield is 87% calculated on the fermentation broth):

| | |
|---|---|
| 13β-ethyl-15α-hydroxy-4-gonene-3,17-dione | 96.7 % |
| 13β-ethyl-4-gonene-3,17-dione | 0.5% |
| other steroids | 1.9% |

### Example 3

### Isolation of 13β-ethyl-11α-hydroxy-4-gonene-3,17-dione

The bioconversion is carried out by known method, for example according to the Hungarian patent 216 874 by fungus *Cylindrocarpon dydimum,* in 5 dm³ useful volume. The amount of the added 13β-ethyl-4-gonene-3,17-dione substrate is 4 g/dm³, the bioconversion is 87%. After terminating the bioconversion the fermentation broth is analyzed and the steroid compounds are found both in the fermentation broth and on the surface of the filtered biomass. The pH of the fermentation broth is adjusted to 3.5-4.0 by addition of 10 % phosphoric acid in order to increase the efficacy of the filtration, 50 g of perfil is added as filtration aid and the temperature of the fermentation broth is increased to 100 °C in order to destroy the microorganism. Then the temperature of the mixture is cooled to 40-45 °C and the biomass is filtered through earthenware filter fitted with technical canvas.

The temperature of the filtrate is cooled to 20-25 °C and mixture is extracted with 2 dm³ of n-propyl acetate. After separating the phases the aqueous phase is repeatedly extracted with 1 dm³ of n-propyl acetate. The organic phases containing the steroid compounds are combined and concentrated under reduced pressure to a volume of about 1 dm³ in a Rotavapor using a water-bath of 65 °C. The organic phase of decreased volume is extracted twice with 0.5 dm³ of 10 % potassium carbonate solution in order to remove the non-steroid impurities. The aqueous phases are discarded after neutralization. 0.2 dm³ of water is added to the organic phase and the organic solvent is evaporated in a Rotavapor using a water-bath of 65 °C. The so obtained crude, steroid containing crystals are filtered and dried.

The biomass is transferred to an apparatus equipped with stirrer and 2 dm³ of 60 w/w % aqueous acetone is added. The mixture is stirred for 30 min and the biomass is filtered through compressive filter. The filtrate is transferred to a Rotavapor and the solvent is distilled off. The residue is cooled to 20 °C and the precipitated steroids containing second crude product is filtered and dried.

The two crude crystalline products obtained by the above processes are transferred into an apparatus equipped with stirrer and dissolved in 0.7 dm³ of 70 w/w % methanol. Then 4 g of Norit and 4 g of perfil are added and the mixture is stirred for 30 min, then the flocculant is filtered through Seitz K5 plate filter. 0.5 dm³ of water is added to the filtrate and the methanol is distilled off in a Rotavapor as described above. The residue is cooled to 20 °C, the precipitated purified homogenous crude crystals are filtered through PO-3 glass filter and dried.

The weight of the so obtained crystals is 18.4 g, and according to HPLC the composition thereof is the following:

| | |
|---|---|
| 13β-ethyl-1α-hydroxy-4-gonene-3,17-dione | 82.1 % |
| 13β-ethyl-4-gonene-3,17-dione | 8.2% |
| other steroids | 4.1% |

In order to separate the desired final product and the starting substrate the above crude product is dissolved in 36 cm³ (the volume is two and a half-fold of the weight of the crystals) of dichloromethane and 180 cm³ of n-hexane is added with stirring from a reservoir slowly over a period of about 1 h. The precipitated crystals are filtered and dried.

The weight of the so obtained 13β-ethyl-11α-hydroxy-4-gonene-3,17-dione is 15.2 g, and according to HPLC the composition thereof is the following (the yield is 83% calculated on the fermentation broth):

| | |
|---|---|
| 13β-ethyl-11α-hydroxy-4-gonene-3,17-dione | 95.2 % |
| 13β-ethyl-4-gonene-3,17-dione | 0.8% |
| other steroids | 2.4% |

### Example 4

### Isolation of 9α-hydroxy-4-androstene-3,17-dione

The bioconversion is carried out by known method, for example according to the US Patent Specification 4 065 236 by bacterium *Mycobacterium fortuitum,* in 5 dm³ useful volume. The amount of the added β-sitosterol substrate is 30 g/dm³ and according to HPLC the concentration of the 9α-hydroxy-4-androstene-3,17-dione in the fermentation broth is 12 g/dm³ when the bioconversion is terminated. After terminating the bioconversion the fermentation broth is analyzed and 1.5 g/dm³ product is found in the fermentation broth and the rest is on the surface of the filtered biomass. The filtered fermentation broth is worked up as described in Example 3, except that the solvent used in the extraction is isobutyl methyl ketone, to yield 6 g crude crystalline product.

The steroids are dissolved from the surface of the biomass with 2 dm³ of 60 w/w % methanol twice and the second crude crystalline product (68 g) is obtained by evaporation of the solvent.

The two crude crystalline products obtained by the above processes are combined and dissolved in 1.1 dm³ of 70 w/w % methanol. Then 8 g of Norit and 8 g of perfil are added and the mixture is stirred for 30 min, then the flocculants are filtered. 0.5 dm³ of water is added to the filtrate and the ethanol is distilled off under reduced pressure. The precipitated purified homogenous crude crystals are filtered and dried.

The weight of the so obtained crystals are 65 g, and according to HPLC the composition thereof is the following:

| | |
|---|---|
| 9α-hydroxy-4-androstene-3,17-dione | 87.2 % |
| other steroids | 8.1% |

The above crude product is dissolved in 0.2 dm³ of dichloroethane and 1 dm³ of n-octane is added with stirring slowly over a period of about 1 h. The precipitated crystals are filtered and dried.

The weight of the so obtained 9α-hydroxy-4-androstene-3,17-dione is 58 g, and according to HPLC the composition thereof is the following (the yield is 92% calculated on the fermentation broth):

| | |
|---|---|
| 9α-hydroxy-4-androstene-3,17-dione | 95.6 % |
| other steroids | 1.6% |

### Example 5

### Isolation of 6ξ-hydroxymethyl-1,4-androstadiene-3,17-dione

### (which is a mixture of 6α and 6ß stereoisomers)

The bioconversion is carried out by known method, for example according to the Hungarian Patent Application No. P07 00584 by bacterium *Arthrobacter simplex,* in 5 dm³ useful volume. The amount of the added 6ξ-hydroxymethyl-4-androstene-3,17-dione substrate is 3 g/dm³ and the bioconversion is 70%. After terminating the bioconversion the fermentation broth is analyzed and the major amount of the steroid compounds is found in the solution, precipitation on the surface of the biomass is only minimal. 50 g of perfil is added as filtration aid to the fermentation broth and the temperature is increased to 100 °C in order to destroy the microorganism. Then the temperature of the mixture is cooled to 30-35 °C and the biomass is filtered through earthenware filter fitted with technical canvas.

The temperature of the filtrate is cooled to 20-25 °C and mixture is extracted with 2 dm³ of ethyl acetate. After separating the phases the aqueous phase is repeatedly extracted with 1 dm³ of ethyl acetate. The organic phases are combined and extracted with 1.2 dm³ of 5 % sodium hydroxide solution in order to remove the non-steroid impurities. The aqueous phases are discarded after neutralization. 0.2 dm³ of water is added to the organic phase and the organic solvent is evaporated in a Rotavapor using a water-bath of 65 °C. The so obtained crude, steroid containing crystals are inhomogeneous, therefore they are dissolved in 0.2 dm³ of methanol and 0.1 dm³ of water and 4 cm³ of 5 % sodium hydroxide solution are added. Then the organic phase is evaporated to yield homogeneous crude crystals.

The weight of the so obtained crystals is 8.8 g, and according to HPLC the composition thereof is the following:

| | |
|---|---|
| 6β-hydroxymethyl-1,4-androstadiene-3,17-dione | 93.91% |
| 6α-hydroxymethyl-1,4-androstadiene-3,17-dione | 0.67% |
| 2,6-bis-hydroxymethyl-1,4-androstadiene-3,17-dione | 0.54% |
| 6ξ-hydroxymethyl-1,4-androstene-3,17-dione | 0.48% |
| 1,4-androstadiene-3,17-dione | 3.56% |

In order to obtain the product of desired purity the removal of the apolar impurities is carried out the following way: the above crude product is dissolved in 20 cm³ of dichloromethane and 100 cm³ of methyl cyclohexane is added with stirring from an addition funel slowly over a period of about 1 h. The precipitated crystals are filtered and dried.

The weight of the so obtained 6ξ-hydroxymethyl-1,4-androstene-3,17-dione is 8.32 g, and according to HPLC the composition thereof is the following (the yield is 77.6% calculated on the fermentation broth):

| | |
|---|---|
| 6β-hydroxymethyl-1,4-androstadiene-3,17-dione | 98.10% |
| 6α-hydroxymethyl-1,4-androstadiene-3,17-dione | 0.43% |
| 2,6-bis-hydroxymethyl-1,4-androstadiene-3,17-dione | 0.49% |
| 6ξ-hydroxymethyl-1,4-androstene-3,17-dione | 0.39% |
| 1,4-androstadiene-3,17-dione | 0.33% |

We note that in the next reaction of the synthesis of exemestane the α and β isomers give the same product, so it is unnecessary to separate them from each other.

### Example 6

### Isolation of 15α-hydroxy-4-androstene-3,17-dione on industrial scale

The bioconversion is carried out according to the method described in Example 1 in 20 m³ useful volume. After terminating the bioconversion the fermentation broth is analyzed and the steroid compounds are found both in the fermentation broth and on the surface of the filtered biomass. The pH of the fermentation broth is adjusted to 3.5-4.0 by addition of 10 % phosphoric acid in order to increase the efficacy of the filtration, and the temperature of the fermentation broth is increased to 100 °C in order to destroy the microorganism. Then the temperature of the mixture is cooled to 40-45 °C, 9 kg/m³ of perfil is added as filtration aid and the biomass is filtered through pressure filter of Lenser type fitted with technical canvas. The weight of the filtered biomass is 700 kg.

The filtrate of the fermentation broth is extracted with 4 m³ of n-propyl acetate in a CINC type separator with countercurrent flow operational method. The raffinate is discarded after neutralization and the organic phase is concentrated to a volume of about 2 m³. The organic phase of decreased volume is extracted twice with 1 m³ of 1 % sodium hydroxide solution in order to remove the non-steroid impurities. The aqueous phases are discarded after neutralization. 1600 dm³ of water is added to the organic phase and the mixture is transferred to an apparatus, which can be heated or cooled and equipped with stirrer and the organic solvent is evaporated. The residue is cooled to 20 °C and the precipitated crude, crystalline product is filtered by C-10 type centrifuge fitted with technical canvas. The so obtained crude crystals contain large amount of polar impurities, therefore they are recrystallized the following way:

In an apparatus, which can be heated or cooled and equipped with stirrer 1300 dm³ of 40 w/w % aqueous ethanol is mixed with the above obtained crude crystals. Then the temperature of the mixture is increased to 65 °C, 5 kg of charcoal and 3 kg of perfil are added and the mixture is stirred for 1 h at this temperature. The flocculants are filtered through Sparkler filter and discarded. The filtrate is transferred into a duplicator and 200 dm³ of water is added and the ethanol is distilled off under atmospheric pressure. The residue is cooled to 20 °C and the precipitated purified crystals are filtered by C-10 type centrifuge fitted with technical canvas. The weight of the so obtained purified crude crystals is 28 kg.

The biomass is transferred to an apparatus, which can be heated or cooled and equipped with stirrer and 8 m³ of 60 w/w % aqueous ethanol is added. Then the temperature of the mixture is increased to 60 °C, the mixture is stirred for 60 min at this temperature and the biomass is filtered by membrane filtering machine. The filtered biomass is incinerated. The filtrate is transferred to a duplicator and the ethanol is distilled off under atmospheric pressure. The residue is cooled to 20 °C and the precipitated crystals are filtered by C-10 type centrifuge fitted with technical canvas. The weight of the crude crystals obtained from the biomass is 135 kg.

The two crude crystalline products obtained by the above processes are transferred into a duplicator equipped with stirrer and dissolved in the mixture of 1000 dm³ of acetone and 1600 dm³ of water at 70 °C. Then 8 kg of sodium carbonate is added and the mixture is refluxed for 1h. Then the solution is cooled to 55 °C, 10 dm³ of acetic acid, 10 kg of charcoal and 5 kg of perfil are added and the mixture is stirred for 60 min, then the flocculants are filtered through Sparkler filter. The filtrate is transferred to a duplicator, 400 dm³ of water is added the acetone is distilled off under atmospheric pressure. The residue is cooled to 20 °C and the precipitated crystals are filtered by C-10 type centrifuge fitted with technical canvas.

The weight of the so obtained homogenous crystals is 112 kg, and according to HPLC the composition thereof is the following:

| | |
|---|---|
| 15α-hydroxy-4-androstene-3,17-dione | 85.0% |
| 4-androstene-3,17-dione | 12.1 % |
| other steroids | 1.8% |

In order to separate the product and the substrate 400 dm³ of methyl cyclohexane is added to the above product in a duplicator and the water is removed from the mixture by azeotropic distillation. Then the precipitated crystals are filtered by C-10 type centrifuge fitted with technical canvas, transferred into an apparatus equipped with stirrer and dissolved in dichloromethane (the volume is twice of the weight of the crystals). Then methyl cyclohexane (the volume is eight-fold of the weight of the crystals) is added with stirring slowly. The precipitated crystals are filtered and dried.

The weight of the so obtained crystalline 15α-hydroxy-4-androstene-3,17-dione is 98 kg, and according to HPLC the composition thereof is the following (the yield is 70% calculated on the fermentation broth):

| | |
|---|---|
| 15α-hydroxy-4-androstene-3,17-dione | 96.2% |
| 4-androstene-3,17-dione | 1.6% |
| other steroids | 1.2% |

When the last crystallization step is repeated - in given case - the amount of the substrate impurity can further be decreased, but in our case it is not necessary for the further chemical steps.

## Claims

1. Process for the selective isolation, purification and separation of monohydroxylated 3,17-diketo-steroid compounds from a solution obtained by microbiological hydroxylation containing a mixture of steroid compounds via selective extraction, purification and selective crystallization of the steroid compounds **characterized by**
a) removing the biomass from the fermentation broth, in given case after treatment with phosphoric acid, by filtration or centrifugation, isolating the steroid compounds from the filtrate with an aliphatic ketone or ester of an aliphatic alcohol, selectively removing the non-steroid type impurities from the organic phase by treatment with an aqueous solution of basic character and obtaining the crude crystalline product containing the hydroxylated steroids by addition of water and evaporation of the solvent and
b) dissolving the steroid compounds from the surface of the filtered biomass obtained in step a) by treatment with C₁-C₄ aliphatic alcohols or ketones or the aqueous solution thereof and obtaining the crude crystalline product containing the hydroxylated steroids after evaporation of the solvent, then
c) dissolving the product obtained in step a) or in given case the mixture of the two crude products obtained in step a) and b) in C₁-C₄ aliphatic alcohol or ketone miscible with water or the aqueous solution thereof, decolorizing the solution and isolating the purified homogeneous crude crystals after evaporation of the solvent and
d) recrystallizing the purified homogeneous crude crystals obtained in step c) from a mixture of chlorinated hydrocarbon/aliphatic or cycloaliphatic hydrocarbon and isolating the pure product.

2. The process according to claim 1 **characterized by** using ethyl acetate, n-propyl acetate or isobutyl methyl ketone as aliphatic ketone or ester of an aliphatic alcohol.

3. The process according to claims 1 or 2 **characterized by** adjusting the basicity and the ionic strength of the aqueous solution used to remove non-steroid impurities by addition of
alkali metal or alkali earth metal hydroxides and/or basic salts thereof, such as carbonates, hydrogen carbonates, phosphates, hydrogen phosphates, acetates.

## Patentansprüche

1. Verfahren zur selektiven Isolierung, Reinigung und Trennen von monohydroxylierten 3,17-Diketosteroid-Verbindungen von einer Lösung, erhalten durch mikrobiologische Hydroxylierung, umfassend eine Mischung von Steroidverbindungen, über selektive Extraktion, Reinigung und selektive Kristallisierung der Steroidverbindungen, **gekennzeichnet durch**
(a) Entfernen der Biomasse von der Fermentationsbrühe im gegebenen Fall nach Behandlung mit Phosphorsäure, **durch** Filtration oder Zentrifugation, Isolieren der Steroidverbindungen vom Filtrat mit einem aliphatischen Keton oder Ester eines aliphatischen Alkohols, selektives Entfernen der Verunreinigungen vom Nicht-Steroidtyp von der organischen Phase **durch** Behandlung mit einer wässrigen Lösung mit basischem Charakter, und Erhalt des rohen kristallinen Produktes, umfassend die hydroxylierten Steroide **durch** Zugabe von Wasser und Verdampfung des Lösungsmittels, und
(b) Auflösen der Steroidverbindungen von der Oberfläche der filtrierten Biomasse, erhalten im Schritt (a), **durch** Behandlung mit aliphatischen C₁-C₄-Alkoholen oder Ketonen oder der wässrigen Lösung davon und Erhalt des rohen kristallinen Produktes, umfassend die hydroxylierten Steroide, nach Verdampfung des Lösungsmittels, anschließendes
(c) Auflösen des Produktes, erhalten im Schritt (a) oder gegebenenfalls der Mischung aus den beiden rohen Produkten, erhalten im Schritt (a) und (b), in aliphatischem C₁-C₄-Alkohol oder Keton, der/das mit Wasser mischbar ist, oder der wässrigen Lösung davon, Entfärben der Lösung und Isolieren der gereinigten homogenen rohen Kristalle nach Verdampfung des Lösungsmittels, und
(d) Rekristallisieren der gereinigten homogenen rohen Kristalle, erhalten im Schritt (c), von einer Mischung aus chloriertem Kohlenwasserstoff/aliphatischem oder cycloaliphatischem Kohlenwasserstoff und Isolieren des reinen Produktes.

2. Verfahren nach Anspruch 1, **gekennzeichnet durch** Verwendung von Ethylacetat, n-Propylacetat oder Isobutylmethylketon als aliphatisches Keton oder Ester eines aliphatischen Alkohols.

3. Verfahren nach den Ansprüchen 1 oder 2, **gekennzeichnet durch** Einstellen der Basizität und der Ionenstärke der wässrigen Lösung, die zur Entfernung der nichtsteroiden Verunreinigungen verwendet wird, **durch** Zugabe von Alkalimetall- oder Erdalkalimetallhydroxiden und/oder basischen Salzen davon wie Carbonaten, Hydrogencarbonaten, Phosphaten, Hydrogenphosphaten, Acetaten.

## Revendications

1. Procédé pour l'isolation sélective, la purification et la séparation de composés 3,17-dicétostéroïdes monohydroxylés à partir d'une solution obtenue par hydroxylation microbiologique contenant un mélange de composés stéroïdes via extraction sélective, purification et cristallisation sélective des composés stéroïdes, **caractérisé par** :
a) l'élimination de la biomasse dans le bouillon de fermentation, le cas échéant après traitement à l'acide phosphorique, par filtration ou centrifugation, l'isolation des composés stéroïdes à partir du filtrat au moyen d'une cétone aliphatique ou d'un ester d'un alcool aliphatique, l'élimination sélective des impuretés non de type stéroïde dans la phase organique par traitement avec une solution aqueuse basique, et l'obtention du produit cristallin brut contenant les stéroïdes hydroxylés par addition d'eau et évaporation du solvant, et
b) la dissolution des composés stéroïdes provenant de la surface de la biomasse filtrée obtenue dans l'étape a), par traitement avec des alcools ou des cétones aliphatiques en C₁ à C₄ ou d'une solution aqueuse de ceux-ci, et l'obtention du produit cristallin brut contenant les stéroïdes hydroxylés après évaporation du solvant, puis
c) la dissolution du produit obtenu dans l'étape a) ou, le cas échant, du mélange des deux produits bruts obtenus dans les étapes a) et b) dans un alcool ou une cétone aliphatique en C₁ à C₄ miscible avec l'eau ou une solution aqueuse de celui-ci, la décoloration de la solution et l'isolation des cristaux bruts homogènes purifiés après évaporation du solvant, et
d) la recristallisation des cristaux bruts homogènes purifiés obtenus dans l'étape c) dans un mélange d'hydrocarbure chloré/hydrocarbure aliphatique ou cycloaliphatique et l'isolation du produit pur.

2. Procédé selon la revendication 1, **caractérisé par** l'utilisation d'acétate d'éthyle, d'acétate de n-propyle ou d'isobutylméthylcétone à titre de cétone aliphatique ou d'ester d'un alcool aliphatique.

3. Procédé selon la revendication 1 ou 2, **caractérisé par** l'ajustement du caractère basique et de la force ionique de la solution aqueuse utilisée pour éliminer les impuretés non stéroïdes par addition d'hydroxydes de métal alcalin ou de métal alcalino-terreux et/ou de sels basiques de ceux-ci, tels que les carbonates, hydrogénocarbonates, phosphates, hydrogéno-phosphates, acétates.
